(19) Europäisches Patentamt
European Patent Office
Office européen des brevets

(11) Publication number: **0 320 075 A1**

# (12) EUROPEAN PATENT APPLICATION

(21) Application number: 88202856.6

(22) Date of filing: 13.12.88

(51) Int. Cl.4: C07D 471/16 , A61K 31/395 , C07D 521/00

(30) Priority: 14.12.87 NL 8703015

(43) Date of publication of application: 14.06.89 Bulletin 89/24

(84) Designated Contracting States: AT BE CH DE ES FR GB GR IT LI LU NL SE

(71) Applicant: DUPHAR INTERNATIONAL RESEARCH B.V
C.J. van Houtenlaan 36
NL-1381 CP Weesp(NL)

(72) Inventor: Hamminga, Derk
c/o OCTROOIBUREAU ZOAN B.V. P.O. Box 140
NL-1380 AC Weesp(NL)
Inventor: Van Wijngaarden, Ineke
c/o OCTROOIBUREAU ZOAN B.V. P.O. Box 140
NL-1380 AC Weesp(NL)
Inventor: Jansen, Johannes W.C.M.
c/o OCTROOIBUREAU ZOAN B.V. P.O. Box 140
NL-1380 AC Weesp(NL)

(74) Representative: Muis, Maarten et al
OCTROOIBUREAU ZOAN B.V. P.O. Box 140
NL-1380 AC Weesp(NL)

## (54) 8,9-Annelated-beta-carbolines and 8,9-annelated-3,4-dihydro-beta-carbolines.

(57) The invention relates to new 8,9-annelated -β-carbolines and 8,9-annelated-3,4-dihydro-β-carbolines having formula 1 or 2

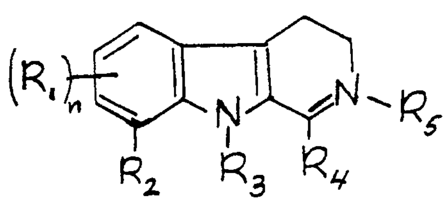

(1)

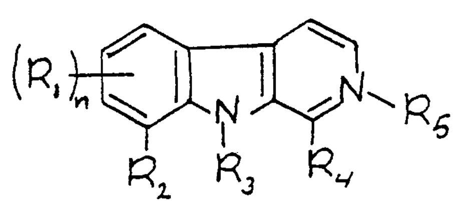

(2)

wherein $R_1$-$R_5$ and n have the meanings given in the specification.

These compounds have good fibrinoltyic activity, and are particularly suitable as oral fibrinolytics.

EP 0 320 075 A1

## 8,9-Annelated-β-carbolines and 8,9-annelated-3,4-dihydro-β-carbolines.

The invention relates to new 8,9-annelated-β-annelated-β-carboline derivatives and 8,9-annelated-3,4-dihydro-β-carboline derivatives and salts and prodrugs thereof, to the preparation of these compounds and to pharmaceutical compositions comprising at least one of these compounds as an active substance. It has been found surprisingly that compounds of the formulae 1 and 2

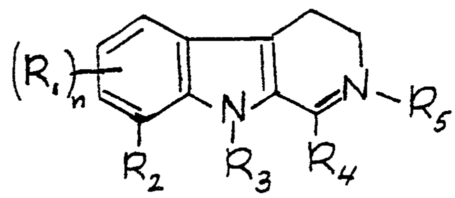

(1)

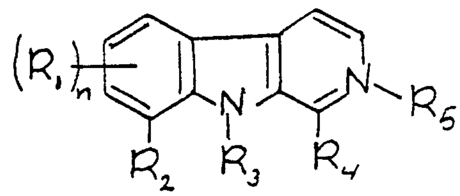

(2)

and the salts and prodrugs thereof have good fibrinolytic properties and may be used in particular as orally active fibrinolytics.

The symbols in the above formulae 1 and 2 have the followings meanings:

$R_1$ is straight or branched alkyl having 1-4 C-atoms, fluorinated or hydroxylated alkyl having 1-4 C-atoms, phenylalkyl having 1-4 C-atoms in the alkyl group, the phenyl group of which may optionally be substituted, or two alkyl groups $R_1$ bound to adjacent carbon atoms together form a ring of 5-7 carbon atoms, or $R_1$ is straight or branched alkenyl or alkynyl having 2-6 C-atoms, which groups may comprise one or more fluorine atoms, or cycloalkyl having 3-6 C-atoms, or $R_1$ is straight or branched alkoxy, alkylthio or alkylsulphonyl having 1-6 C-atoms which may be substituted with one or more fluorine atoms or hydroxyl groups, or with one optionally substituted phenyl group, or two alkoxy groups and/or alkylthio groups bound to adjacent carbon atoms may form a ring which consists of 5-7 ring atoms and which may be substituted with hydroxymethyl, alkoxy or alkoxyalkyl groups having 1-6 C-atoms, or $R_1$ is a cycloalkoxy group or a cycloalkylthio group having 3-6 C-atoms, or $R_1$ is straight or branched alkoxy-, alkylthio- or alkylsulphonylalkyl having 26 C-atoms, or $R_1$ is an alkoxycarbonylmethyl group having 1-4 C-atoms in the alkoxy group, or $R_1$ is a group of $R_6R_7N\text{-}CO\text{-}CH_2\text{-}$, $R_6R_7N\text{-}CO\text{-}$, $R_6R_7N\text{-}SO_2\text{-}CH_2$ or $R_6R_7N\text{-}SO_2\text{-}$, wherein $R_6$ and $R_7$ independently of each other are hydrogen, alkyl having 1-3 C-atoms, or together with the nitrogen atom form a heterocyclic 5- or 6-ring, or $R_1$ is hydroxy, halogen, cyano, straight or branched alkoxycarbonyl having 1-6 C-atoms in the alkoxy group, cycloalkylsulphonyl having 3-8 C-atoms;

n has the value 0-2;

$R_2$ + $R_3$ together with the carbon atom and the nitrogen atom to which they are bound and the intermediate carbon atom, form a heterocyclic group which consists of 5-10 ring atoms and which, besides the nitrogen atom already present, may comprise a second hetero atom from the group N, O, S, S-O or $SO_2$, and which may be substituted with alkyl groups having 1-4 C-atoms which may form a spiroalkyl group having 2-5 C-atoms, or the hetero group may be substituted with an optionally substituted phenyl group, or the ring formed by $R_2$ + $R_3$ may be annelated with a saturated or unsaturated carbocyclic or heterocyclic ring which optionally consists of 5- or 6- ring atoms and which may be substituted;

$R_4$ is hydrogen, straight or branched alkyl having 1-8 C-atoms, alkoxy- or alkylthioalkyl having 2-8 C-atoms, alkenyl or alkenyl having 2-8 C-atoms, which groups may be substituted with one or more fluorine atoms or hydroxy groups or with a cycloalkyl group having 3-7 C-atoms or with a phenyl group which is optionally substituted with one or two groups $R_8$, which independently of each other are alkyl or alkoxy having 1-4 C-atoms, halogen, trifluoromethyl, cyano, or hydroxy, and two alkyl groups or alkoxy groups $R_8$ bound to adjacent C-atoms may form a cyclic group of 5-7 ring atoms which is annelated with the phenyl group, or $R_4$ is cycloalkyl having 3-8 C-atoms which may be substituted with one or more fluorine atoms, alkyl groups or cycloalkyl groups which may be annelated with a benzene ring, or $R_4$ is cycloalkenyl having 5-7 C-atoms which may be substituted with methyl groups, or $R_4$ is a bridged hydrocarbon group, or $R_4$ is straight or branched alkoxycarbonylalkyl having 0-6 C-atoms in the alkoxy group and 1-3 C-atoms in the alkyl group, or $R_4$ is a group $R_6R_7N\text{-}CO\text{-}R_9\text{-}$ or $R_6R_7N\text{-}SO_2\text{-}R_9\text{-}$, wherein $R_6$ and $R_7$ have the above-mentioned meanings and $R_9$ is alkyl having 1-3 C-atoms, or $R_4$ is alkylsulphonylalkyl having 1-3 C-atoms per alkyl group, or a phenylsulphonylalkyl group having 1-3 C-atoms in the alkyl group and 0-2 groups $R_8$ in the

phenyl group, or $R_4$ is a phenyl group substituted with 0-4 groups $R_{10}$, wherein groups $R_{10}$ independently of each other are straight or branched alkyl having 1-6 C-atoms which may be substituted with one or more fluorine atoms or hydroxyl groups, or with one cyano group, or with straight or branched alkoxycarbonyl having 0-6 C-atoms in the alkoxy group, or with a group $R_6R_7N$-CO- or $R_6R_7N$-$SO_2$- wherein $R_6$ and $R_7$ have the above-mentioned meanings or two groups $R_{10}$ bound to adjacent carbon atoms form a carbocyclic ring which consists of 5-7 ring atoms and is annelated with the phenyl group, or $R_{10}$ is straight or branched alkyl(1-4C)-oxy-alkyl(0-3C), alkyl(1-4C)-thio-alkyl(0-3C) or alkyl(1-4C)-sulphonyl-alkyl(0-3C), which groups may comprise one or more fluorine atoms or hydroxyl groups or of which two alkoxy groups or alkylthio groups bound to adjacent carbon atom form a ring consisting of 5-7 ring atoms, or $R_{10}$ is cycloalkoxy, cycloalkylthio or cycloalkylsulphonyl having 3-6 C-atoms, or $R_{10}$ is cycloalkyl having 3-6 C-atoms, or $R_{10}$ is straight or branched alkoxycarbonyl having 1-6 C-atoms in the alkoxy group, a group $R_6R_7N$-CO- or $R_6R_7N$-$SO_2$ wherein $R_6$ and $R_7$ have the above-mentioned meanings, or $R_{10}$ is halogen or hydroxy, or $R_4$ is a monocyclic or bicyclic heteroaryl group in which N and/or O and/or S may be present as hetero atoms;
$R_5$ is absent or $R_5$ is alkyl (having an anion as a counter ion), or oxygen.

Suitable acids with which the compounds of formula 1 and 2 according to the invention can form pharmaceutically acceptable acid addition salts are, for example, hydrochloric acid, sulphuric acid, phosphoric acid, nitric acid, and organic acids, for example, citric acid, fumaric acid, maleic acid, tartaric acid, acetic acid, benzoic acid, p-toluene sulphonic acid, methane sulphonic acid, and the like.

When $R_2$ and $R_3$ together with the carbon atom and nitrogen atom to which they are bound and the intermediate carbon atom form a ring which is substituted, the compounds of formulae 1 and 2 may comprise one or more chiral centres. The invention relates to both racemates and individual enantiomers.

The invention also relates to prodrugs of the compounds of formulae 1 and 2, i.e. derivatives of these compounds which as such are inactive, from which, after splitting off an easily removable group, for example, an ester group or an ether group, an active compound of formulae 1 and 2 is formed.

The carboline derivatives according to the invention are orally active fibrinolytics and may hence be used in controlling already formed venous or arterial thrombi or may be administered to prevent thrombi. The compounds may be used, for example for a short period of time during operations or for a long period of time in the case of increased risk after, for example, myocard infarct, cerebral or peripheral suffering. The best compounds possibly operate via an enhancement of the tissue plasminogen activator activity, as a result of which the possibility of spontaneous bleedings can be prevented.

The oral fibrinolytic activity of the compounds according to the invention was established in the first instance in rats in the so-called DBCLT (Diluted Blood Clot Lysis Test; Taylor F.B. et al, Fed. Proc. (1981), 40, 2092-2098). Rats are treated orally with the compound to be tested. After 1-3 hours blood is taken. $^{125}$I-labelled fibrinogen and thrombin are added so that a blood clot is formed which, depending on the extent of fibrinolytic activity caused by the compound to be tested, dissolves more rapidly compared with blood clots of untreated animals.

The enhancement of tissue plasminogen activator activity was measured in cultures of endothelial cells (Thrombosis Diathesis Haemorrhagis (Stuttgart), 34, (1975), pp. 825-839; and Thrombosis and Haemostasis, 51, 91984), p. 392).

The pharmacologically active compounds belonging to the invention, their salts and prodrugs can be brought into forms suitable for administration, for example, pills, tablets, coated tablets, capsules, powders, injection liquids, and the like, by means of the techniques conventionally used for this purpose and while using suitable auxiliary substances, for example, solid or liquid carrier materials.

The dosage in which the compounds according to the invention may be used depend on the severity and the nature of the disease to be treated and on the way of administration.

The compounds of formula 1, wherein $R_1$-$R_5$ and n have the above-mentioned meanings can be prepared in at least one of the following manners. For example, the compounds of formula 1, wherein the substituent $R_5$ is not present, may be prepared

a) by a Bischler-Napieralski cyclisation of a compound of formula 3

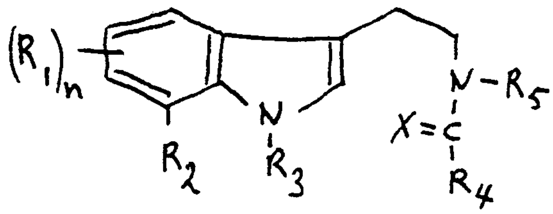

(3)

wherein $R_1$-$R_1$, and n, have the meanings mentioned in formula 1, X is oxygen or sulphur and $R_5$ is hydrogen. Suitable methods for Bischler-Napieralski cyclisations are described inter alia in Organic Reactions vol. VI, p. 74.

The starting compounds of formula 3 required for this mode of preparation can be obtained in a manner known for the synthesis of analogous compounds, for example, by reaction of a compound of formula 4

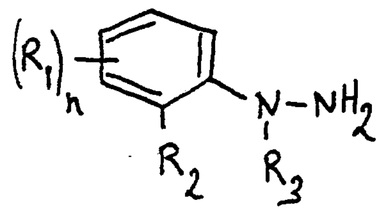

(4)

with 4-chlorobutyraldehyde or 4-aminobutyraldehyde diethylacetal.

The intermediate products of formula 5 obtained in this manner

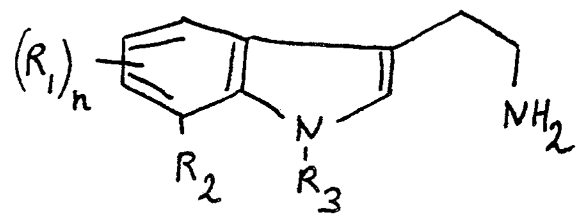

(5)

can be converted into the analogous starting compounds of formula 3 by reacting them in a manner known per se with a compound of formula $R_4$-CX-Y, wherein X is oxygen or sulphur and Y is a reactive group, for example halogen, ethoxycarbonyl, etc. The starting compounds of formula 3 wherein X is sulphur can also be prepared in a manner known per se by reaction of the corresponding compounds, wherein X is oxygen, with a suitable sulphurating agent, for example, phosphorpentasulphide or with the Lawesson's reagent, preferably in a suitable solvent at temperatures between 20 and 180 °C.

b) by oxidation of a compound of formula 6 or a salt thereof

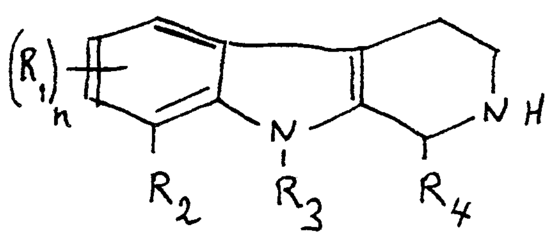

(6)

wherein $R_1$, $R_2$, $R_3$, $R_4$ and n have the above-mentioned meanings with a suitable oxidation agent. The intermediate compounds of formula 6 also have interesting pharmacological properties, in particular fibrinolytic activity. The compounds of formula 1 can be prepared in a good yield in particular by oxidation with potassium permanganate as described, for example, in Synthesis, Synthetic Communications (1985), pp. 1134-1135. The reaction is preferably carried out under an atmosphere of nitrogen in a suitable solvent, for example, tetrahydrofuran, dioxan, etc., at temperatures from -10 to 20 °C. The starting compounds of formula 6 required for this mode of preparation can be obtained analogously to the method described in Advances in Heterocyclic Chemistry, vol. 3, pp. 79-207 (The Carbolines). More in particular, compounds of this type can be obtained in a good yield by reaction of a compound of formula 5 or a salt thereof, wherein $R_1$, $R_2$, $R_3$ and n have the above-mentioned meanings, with an aldehyde of formula 7

$$R_4 - \overset{\overset{\displaystyle O}{\|}}{C} - H \qquad (7)$$

wherein $R_4$ has the above-mentioned meaning. The reaction is preferably carried out in a suitable solvent, for example, acetic acid, alcohol, etc., at a temperature between 10 and 120 °C.

The compounds of formula 1, wherein $R_5$ is alkyl, and wherein $R_1$, $R_2$, $R_3$, $R_4$ and n have the above-mentioned meanings can be prepared in an analogous manner as described in Chem. Pharm. Bull. 33, (8), 3237-3249 (1985), for example, by reaction of a compound of formula 3, wherein $R_1$, $R_2$, $R_3$, $R_4$ and n have the above-mentioned meanings and wherein X is sulphur and $R_5$ is alkyl, with a benzylhalide, preferably in a suitable solvent, for example, acetonitrile. The starting compounds of formula 3 required for this reaction can also be obtained by reacting an analogous compound of formula 3, wherein X is oxygen, with a suitable sulphurating agent, for example, with phosphorpentasulphide or with the Lawessoon's reagent, preferably in a suitable solvent, for example, xylene, acetonitrile, etc., at temperatures between 20 and 180° C.

The compound of formula 2, wherein $R_1$, $R_2$, $R_3$, $R_4$, $R_5$ and n have the meanings mentioned in formula 2 can be prepared in at least one of the following manners.

Compounds of formula 2, wherein the substituent $R_5$ is absent, can be obtained 1) by dehydrogenating a compound of formula 1 or 6, wherein $R_1$, $R_2$, $R_3$, $R_4$ and n have the meanings mentioned in formulae 1 and 6, in the presence of a suitable catalyst, for example palladium on carbon, preferably in a suitable solvent, for example xylene, at temperatures from 80-220° C, or 2) by conversion with sulphur, preferably in a suitable solvent, for example, xylene, at temperatures from 80-220° C, or 3) by oxidation with a suitable oxidant, for example, potassium permangana te preferably in a suitable solvent, for example, tetrahydrofuran or dioxan at temperatures between -10 and 100° C.

The compounds of formulae 1 and 2, wherein $R_5$ is alkyl and wherein $R_1$, $R_2$, $R_3$, $R_4$ and n have the meanings mentioned in formulae 1 and 2, can also be obtained by reaction of a compound of formula 1 and of a compound of formula 2, respectively, wherein $R_5$ is absent, with a compound of formula $R_5$-X, wherein $R_5$ is alkyl and wherein X is a group or atom which may be replaced by a nucleophile, for example, halogen, an alkyl-$OSO_2$-O group, etc. The reaction is preferably carried out in a suitable solvent, for example, acetonitrile, tetrahydrofuran, at temperatures from 10-120° C.

The compounds of formula 1 and 2, wherein $R_5$ is oxygen and wherein the other symbols have the meanings mentioned in formulae 1 and 2, can be prepared, for example, by oxidation of the analogous compounds of formulae 1 and 2, wherein $R_5$ is absent, with a suitable oxidant, for example, perbenzoic acid, peracetic acid, hydrogen peroxide, etc., preferably in a suitable solvent, for example, chloroform, acetic acid or water, at temperatures from 0-100° C.

The invention will now be described in more detail with reference to the ensuing specific examples.

## EXAMPLE I

**8-Phenyl-4,4-dimethyl-5,6,10,11-tetrahydro-4H-pyrido[4′,3′:4,5]pyrrolo-[3,2,1-ij]-quinoline hydrochloride**

0.5 g (1.57 mmol of 1-[2-(benzoylamino)-ethyl]-6,6-dimethyl-5,6-dihydro-4H-pyrrolo-[3,2,1-i,j]-quinoline are added at 20° C to 3 ml of phosphoroxychloride. The mixture is then refluxed for one hour, cooled and, while cooling, poured into 20 ml of 1N sodium hydroxide solution. The mixture is extracted with methylene chloride, washed with water, dried and evaporated under reduced pressure. In this manner 0.47 g (100% of the free base are obtained. The free base is dissolved in alcohol and alcoholic hydrochloric acid is added to this solution. This solution is then evaporated under reduced pressure. The residue is stirred for 24 hours with ether/petroleum ether. The solid is sucked off and washed with petroleum ether and dried. Yield: 0.55 g (100%); melting-point 271-273° C.

In a similar way the following compounds were obtained:

(1) 8-cyclohexyl-5,6,10,11-tetrahydro-4H-pyrido-[4′, 3′:4,5]pyrrolo[3,2,1-ij]-quinoline hydrochloride; yield 62%, melting-point 193-195° C;

(2) 10-cyclopentyl-5,6,7,8,12,13-hexahydro-4H-pyrido[4′,3′:4,5]pyrrolo[3,2,1-kl]-1-benzazocine hydrochloride; yield 43%, melting-point 259-259.5° C.

## EXAMPLE II

**9-Phenyl-4,5,6,7,11,12-hexahydro-pyrido[4′,3′:4,5]-pyrrolo-[3,2,1-j,k]-1-benzazepine hydrochloride**

1.17 g (3.9 mmol) of 9-phenyl-4,5,6,7,9,10,11,12-octahydropyrido-[4′,3′:4,5]-pyrrolo-[3,2,1-j,k]-1-benzazepine are dissolved in 78 ml of dry tetrahydrofuran and cooled to 0° C under a nitrogen atmosphere. At this temperature 9.8 g (62 mmol) of potassium permanganate powder are added in 50 minutes. Stirring at 0° C is then continued for 1 hour. The mixture is then filtered over hyflo and evaporated under reduced pressure. The residue is chromatographed over silicagel (using methylene chloride to which 7% by volume of methanol and 0.5% by volume of ammonia has been added as an eluent). The purified substance is dissolved in ethyl acetate, after which 1,1 equivalent of ethanolic hydrochloric acid is added. The formed salt is filtered off, washed and dried. Yield: 0.96 g (74%); melting-point 227-228° C.

In an analogous manner the following compounds were obtained:

(1) 2-chloro-8-phenyl-5,6,10,11-tetrahydro-4H-pyrido[4′,3′:4,5]pyrrolo[3,2,1-ij]quinoline hydrochloride; yield 56%, melting-point 256-257° C;

(2) 10-cyclohexyl-5,6,7,8,12,13-hexahydro-4H-pyrido-[4′,3′:4,5]pyrrolo[3,2,1-kl]-1-benzazocine hydrochloride; yield 61%, melting-point 259-260° C;

(3) 10-(4-methylphenyl)-5,6,7,8,12,13-hexahydro-4H-pyrido[4′,3′:4,5]pyrrolo[3,2,1-kl]-1-benzazocine hydrochloride; yield 48%, melting-point 240-242° C.

(4) 11-cyclohexyl-4,5,6,7,8,9,13,14-octahydro-pyrido[4′,3′:4,5]pyrrolo[3,2,1-1m]-1-benzazonine hydrochloride; $^{13}$C-NMR analysis:

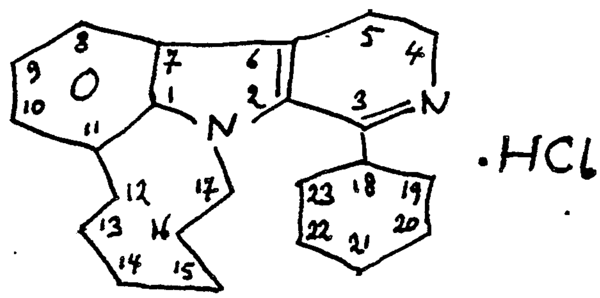

| | | | | | | | | |
|---|---|---|---|---|---|---|---|---|
| 1 | 138.82 | S | 9 | 128.58 | D | 17. | 45.25 | T |
| 2 | 131.05 | S | 10 | 120.49 | D | 18 | 43.52 | D |
| 3 | 165.99 | S | 11 | 125.60 | S | 19 | 31.37 | T |
| 4 | 47.02 | T | 12 | 33.69 | T | 20 | 26.54 | T |
| 5 | 19.88 | T | 13 | 32.32 | T | 21 | 26.02 | T |
| 6 | 121.80 | S | 14 | 26.76 | T | 22 | 26.54 | T |
| 7 | 128.42 | S | 15 | 24.22 | T | 23 | 31.37 | T |
| 8 | 117.83 | D | 16 | 33.09 | T | | | |

## EXAMPLE III

9-(4-Methylphenyl)-4,5,6,7-tetrahydro-pyrido[4′,3′:5]pyrrolo[3,2,1-jk]-1-benzazepine-hydrochloride

2.5 g (7.9 mmol) of 9-(4-methylphenyl)-4,5,6,7,9,10,12-octahydro-pyrido-[4′,3′:4,5]-pyrrolo-[3,2,1-jk]-1-benzazepine are stirred at 140° C for 16 hours together with 0.76 g (24 mmol) of sulphur in 20 ml of xylene. The mixture is then evaporated in vacuo. The residue is chromatographed over silicagel, first with methylene chloride and then with a mixture of methylene chloride - methanol = 95:5 as an eluent. The product thus purified is dissolved in ethyl acetate, after which 1.1 equivalent of alcoholic hydrochloric acid are added. The solid is sucked off, washed and dried. Yield: 0.7 g (25%); melting-point 241-244° C.

In the same manner the following compound was prepared:

10-cyclohexyl-5,6,7,8-tetrahydro-4H-pyrido[4′3′:4,5]-pyrrolo[3,2,1-kl]-1-benzazocine hydrochloride; yield 24%, melting-point 255° C (decomposition).

EXAMPLE IV

6,6-Dimethyl-9-(4-methylphenyl)-4,5,6,7-tetrahydropyrido[4′,3′:4,5]pyrrolo[3,2,1-jk]-1-benzazepine hydrochloride

6.1 g (17.7 mmol) of 6,6-dimethyl-9-(4-methylphenyl)-4,5,6,7,9,10,11,12-octahydro-pyrido[4′,3′:4,5]-pyrrolo[3,2,1-jk]-1-benzazepine were dissolved in 365 ml of tetrahydrofuran, cooled to 0° C, and at this temperature 35.1 g (0.22 mol) of potassium permanganate powder were added in 1 hour. The mixture was stirred for 72 hours at room temperature, and filtered over hyflo. The filtrate was evaporated and the residue was chromatographed over silicagel using methylene chloride/methanol (98/2) as eluent. The desired fractions were evaporated, and the residue was dissolved in ethyl acetate. After addition of alcoholic hydrochloric acid the formed hydrochloride was sucked off, washed with ethyl acetate and dried. Yield 2.0 g (33%), melting-point 266-267° C.

EXAMPLE V

10-(4-Methylphenyl)-5,6,7,8-tetrahydro-4H-pyrido-[4′,3′:4,5]pyrrolo[3,2,1-kl]-1-benzazocine

4.3 g (13 mmol) of 10-(4-methylphenyl)-5,6,7,8,12,13-hexahydro-4H-pyrido[4′,3′:4,5]pyrrolo[3,2,1-kl;]-1-benzazocine were stirred with palladium (10%) on carbon in 70 ml of toluene at boiling temperature for 72 hours. The reaction mixture was filtered, the filtrate was evaporated, and the residue was chromatographed over silicagel using ether-petroleum ether (1/1) as eluent. The desired fractions were evaporated. Yield 3.6 g (84%), melting-point 177.5-178° C.

In a similar manner the following compounds were prepared:

(1) 8-cyclohexyl-5,6-dihydro-4H-pyrido[4′,3′:4,5]pyrrolo[3,2,1-ij]-quinoline hydrochloride; yield 94%, melting-point 305-307° C;

(2) 10-(4-methylphenyl)-1,2-dihydro-pyrido[4′,3′:4,5]pyrrolo[3,2,1-de][1,4]benzoxazine hydrochoride; yield 55%, melting-point 298-291° C.

EXAMPLE VI

10-(4-Methylphenyl)-5,6,7,8-tetrahydro-4H-pyrido-[4′,3′:4,5]pyrrolo[3,2,1-kl]-1-benzazocine 11-oxide

2.6 (8 mmol) of 10-(4-methylphenyl)-5,6,7,8- tetrahydro-4H-pyrido[4′,3′:4,5]pyrrolo[3,2,1-kl]-1-benzazocine were dissolved in 200 ml of chloroform, 1.5 g (8.7 mmol) of m-chloroperbenzoic acid were added, and the mixture was stirred for 20 hours. The reaction mixture was washed twice with sodium bisulfite solution and twice with 2N sodium hydroxide, dried and evaporated. The residue was purified by means of flash chromatography using methylene chloride/methanol (95/5) as eluent. The desired fractions were evaporated in vacuo. Yield 2.8 g (95%), melting-point 256-257° C.

In a similar manner the following compounds were prepared:

(1) 8-cyclohexyl-5,6-dihydro-4H-pyrido[4′,3′:4,5]pyrrolo[3,2,1-ij]-quinoline 9-oxide; yield 84%, melting-point 203-206° C.

(2) 9-(4-methylphenyl)-4,5,6,7-tetrahydro-pyrido-[4′,3′:4,5]pyrrolo[3,2,1-jk]-1-benzazepine 10-oxide; yield 57%, melting-point 233-236° C.

Preparation of intermediate compounds

11-cycohexyl-4,5,6,7,8,9, 11,12,13,14-decahydro-pyrido[4′,3′:4,5]pyrrolo[3,2,1-1m]-1-benzazonine hydrochloride

a) 15.0 g (85.1 mmol) of 2,3,4,5,6,7-hexahydro-1H-1-benzazonine were added to a mixture of 14 ml of concentrated sulfuric acid in 53 ml of water and 35 ml of methylene chloride under an atmosphere of nitrogen. The mixture was cooled to -5° C, and at this temperature a solution of 5 g (85.1 mmol) of sodium nitrite in 37 ml of water were added dropwise, while stirring, such that the temperature remained below 0° C. The mixture was stirred for 1 hour at 0° C, the methylene chloride layer was separated, and evaporated in vacuo. Yield 17.2 g (98%) of a brown oil.

b)17.0 g (83.3 mmol) of this product were dissolved in 78 ml of tetrahydrofuran, and this solution was added dropwise (under nitrogen) to a boiling suspension of 6.3 g (166.6 mmol) of lithium aluminiumhydride in 157 ml of tetrahydrofuran. The mixture was then boiled for 1 hour. After cooling 6.3 ml of water, 12.6 ml of 2N sodium hydroxide and 12.6 ml of water were added dropwise. The mixture was boiled for 15 minutes, cooled and filtered. The filtrate was evaporated in vacuo. Yield 15.3 g of crude 1-amino-2,3,4,5,6,7-hexahydro-1H-1-benzazonine, which was used in the following reaction step without further purification.

c) The crude product (15.3 g) was dissolved in a mixture of 160 ml of methanol and 16 ml of water. After addition of 9.4 g of 4-chlorobutyraldehyde the mixture was boiled for 16 hours, and evaporated in vacuo. The residue was shaken with a mixture of methylene chloride and 2N sodium hydroxide. The methylene chloride layer was separated and evaporated in vacuo. The residue was purified by means of flash chromatography using methylene chloride/methano/ammonia (92/7.5/2.5) as eluent. After evaporating the desired fractions 11.0 g of 1-(2-aminoethyl)-4,5,6,7,8,9-hexahydro-pyrrolo[3,2,1-1m]-1- benzazonine were obtained.

d)1.5 g (6.2 mmol) of the obtained product together with 0.69 g (6.2 mmol) of cyclohexyl carboxaldehyde were dissolved in 30 ml of acetic acid, and heated at 40° C for 48 hours. The mixture was then evaporated in vacuo and shaken with methylene chloride and 2N sodium hydroxide. The methylene chloride layer was separated and chromatographed over silicagel using methylene chloride/methanol (95/5) as eluent. The desired fractions were evaporated in vacuo, and the residue was dissolved in ethyl acetate. After adding alcoholic hydrochloric acid the above-mentioned hydrochloride (1.51 g; 65%) was obtained having a melting-point of 253-254° C.

In a similar way the following compounds were prepared:

(1) 2-chloro-8-phenyl-5,6,8,9,10,11-hexahydro-4H-pyrido[4′,3′:4,5]pyrrolo[3,2,1-ij]-quinoline hydrochloride; melting-point 295-297° C;

(2) 9-phenyl-4,5,6,7,9,10,11,12-octahydro-pyrido[4′,3′:4,5]pyrrolo[3,2,1-jk]-1-benzazepine hydrochloride; melting-point 282-285° C;

(3) 9-(4-methylphenyl)-4,5,6,7,9,10,11,12-octahydropyrido[4′,3′:4,5]pyrrolo[3,2,1-jk]-1-benzazepine hydrochloride; melting-point 262-266° C;

(4) 6,6-dimethyl-9-(4-methylphenyl-4,5,6,7,9,10,11,12-octahydro-pyrido[4′,3′:4,5]pyrrolo[3,2,1-jk]-1-benzazepine hydrochloride; melting-point 286-287° C;

(5) 10-cyclohexyl-5,6,7,8,10,11,12,13-octahydro-4H-pyrido[4′,3′:4,5]pyrrolo[3,2,1-kl]-1-benzazocine hydrochloride; melting-point 278-280° C;

(6) 10-(4-methylphenyl)-5,6,7,8,10,11,12,13-octahydro-4H-pyrido[4′3′:4,5]pyrrolo[3,2,1-kl]-1-benzazocine hydrochloride; melting-point 268-270° C.

## Claims

1. Compounds of formula 1 or 2

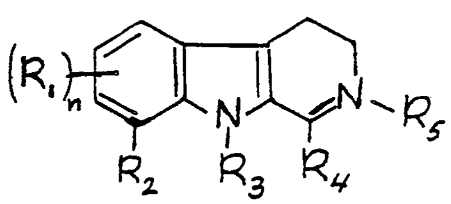

(1)

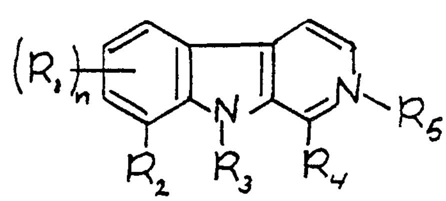

(2)

wherein the symbols have the following meanings:

$R_1$ is straight or branched alkyl having 1-4 C-atoms, fluorinated or hydroxylated alkyl having 1-4 C-atoms, phenylalkyl having 1-4 C-atoms in the alkyl group, the phenyl group of which may optionally be substituted, or two alkyl groups $R_1$ bound to adjacent carbon atoms together form a ring of 5-7 carbon atoms, or $R_1$ is straight or branched alkenyl or alkynyl having 2-6 C-atoms, which groups may comprise one or more fluorine atoms, or cycloalkyl having 3-6 C-atoms, or $R_1$ is straight or branched alkoxy, alkylthio or alkylsulphonyl having 1-6 C-atoms which may be substituted with one or more fluorine atoms or hydroxyl groups, or with one optionally substituted phenyl group, or two alkoxy groups and/or alkylthio groups bound to adjacent carbon atoms may form a ring which consists of 5-7 ring atoms and which may be substituted with hydroxymethyl, alkoxy or alkoxyalkyl groups having 1-6 C-atoms, or $R_1$ is a cycloalkoxy group or a cycloalkylthio group having 3-6 C-atoms, or $R_1$ is straight or branched alkoxy-, alkylthio- or alkylsulphonylalkyl having 2-6 C-atoms, or $R_1$ is an alkoxycarbonylmethyl group having 1-4 C-atoms in the alkoxy group, or $R_1$ is a group $R_6R_7N\text{-}CO\text{-}CH_2\text{-}$, $R_6R_7N\text{-}CO\text{-}$, $R_6R_7N\text{-}SO_2\text{-}CH_2$ or $R_6R_7N\text{-}SO_2\text{-}$, wherein $R_6$ and $R_7$ independently of each other are hydrogen, alkyl having 1-3 C-atoms, or together with the nitrogen atom form a heterocyclic 5- or 6-ring, or $R_1$ is hydroxy, halogen, cyano, straight or branched alkoxycarbonyl having 1-6 C-atoms in the alkoxy group, cycloalkylsulphonyl having 3-8 C-atoms;

n has the value 0-2;

$R_2$ + $R_3$ together with the carbon atom and the nitrogen atom to which they are bound and the intermediate carbon atom, form a heterocyclic group which consists of 5-10 ring atoms and which, besides the nitrogen atom already present, may comprise a second hetero atom from the group N, O, S, S-O or $SO_2$, and which may be substituted with alkyl groups having 1-4 C-atoms which may form a spiroalkyl group having 2-5 C-atoms, or the hetero group may be substituted with an optionally substituted phenyl group, or the ring formed by $R_2$ + $R_3$ may be annelated with a saturated or unsaturated carbocyclic or heterocyclic ring which optionally consists of 5- or 6-ring atoms and which may be substituted;

$R_4$ is hydrogen, straight or branched alkyl having 1-8 C-atoms, alkoxy- or alkylthioalkyl having 2-8 C-atoms, alkenyl or alkynyl having 2-8 C-atoms, which groups may be substituted with one or more fluorine atoms or hydroxy groups or with a cycloalkyl group having 3-7 C-atoms or with a phenyl group which is optionally substituted with one or two groups $R_8$, which independently of each other are alkyl or alkoxy having 1-4 C-atoms, halogen, trifluoromethyl, cyano, or hydroxy, and two alkyl groups or alkoxy groups $R_8$ bound to adjacent C-atoms may form a cyclic group of 5-7 ring atoms which is annelated with the phenyl group, or $R_4$ is cycloalkyl having 3-8 C-atoms which may be substituted with one or more fluorine atoms, alkyl groups or cycloalkyl groups which may be annelated with a benzene ring, or $R_4$ is cycloalkenyl having 5-7 C-atoms which may be substituted with methyl groups, or $R_4$ is a bridged hydrocarbon group, or $R_4$ is straight or branched alkoxycarbonylalkyl having 0-6 C-atoms in the alkoxy group and 1-3 C-atoms in the alkyl group, or $R_4$ is a group $R_6R_7N\text{-}CO\text{-}R_9\text{-}$ or $R_6R_7N\text{-}SO_2\text{-}R_9\text{-}$, wherein $R_6$ and $R_7$ have the above-mentioned meanings and $R_9$ is alkyl having 1-3 C-atoms, or $R_4$ is alkylsulphonylalkyl having 1-3 C-atoms per alkyl group, or a phenylsulphonylalkyl group having 1-3 C-atoms in the alkyl group and 0-2 groups $R_8$ in the phenyl group, or $R_4$ is a phenyl group substituted with 0-4 groups $R_{10}$, wherein groups $R_{10}$ independently of each other are straight or branched alkyl having 1-6 C-atoms which may be substituted with one or more fluorine atoms or hydroxyl groups, or with one cyano group, or with straight or branched alkoxycarbonyl having 0-6 C-atoms in the alkoxy group, or with a group $R_6R_7N\text{-}CO\text{-}$ or $R_6R_7N\text{-}SO_2\text{-}$ wherein $R_6$ and $R_7$ have the above-mentioned meanings or two groups $R_{10}$ bound to adjacent carbon atoms form a carbocyclic ring which consists of 5-7 ring atoms and is annelated with the phenyl group, or $R_{10}$ is straight or branched alkyl-(1-4C)-oxy-alkyl(0-3C), alkyl(1-4C)-thio-alkyl(0-3C) or alkyl(1-4C)-sulphonyl-alkyl(0-3C), which groups may comprise one or more fluorine atoms or hydroxyl groups or of which two alkoxy groups or alkylthio groups bound to adjacent carbon atom form a ring consisting of 5-7 ring atoms, or $R_{10}$ is cycloalkoxy, cycloalkylthio or cycloalkylsulphonyl having 3-6 C-atoms, or $R_{10}$ is cycloalkyl having 3-6 C-atoms, or $R_{10}$ is straight or branched alkoxycarbonyl having 1-6 C-atoms in the alkoxy group, a group $R_6R_7N\text{-}CO\text{-}$ or $R_6R_7N\text{-}SO_2$ wherein $R_6$ and $R_7$ have the above-mentioned meanings, or $R_{10}$ is halogen or hydroxy, or $R_4$ is a monocyclic or bicyclic heteroaryl group in which N and/or O and/or S may be present as hetero atoms;

$R_5$ is absent or $R_5$ is alkyl (having an anion as a counter ion), or oxygen.

and salts and prodrugs thereof.

2. Pharmaceutical compositions having fibrinolytic activity which comprise at least one compound as claimed in Claim 1 as an active substance.

3. A method of dissolving blood clots, characterized in that a composition as claimed in Claim 2 is used.

4. A method of preparing carboline derivatives as claimed in claim 1, characterized in that

a) a compound of formula 3

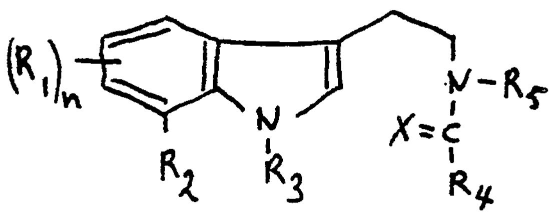

(3)

is cyclised according to Bischler-Napieralski, or

b) a compound of formula 6

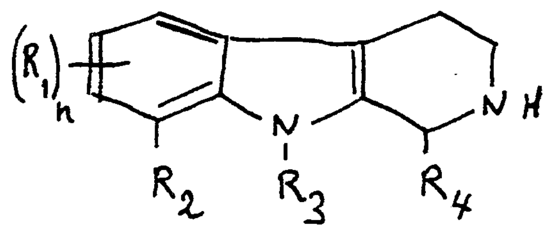

(6)

is oxidised; or

c) a compound of formula 3 is converted with benzylhalide; or

d) a compound of formula 1 or 6 i) is dehydrogenated in the presence of a suitable catalyst to form a compound of formula 2; or ii) is converted with sulphur; or iii) is oxidised with a suitable oxidant, for example, potassium permanganate; or

e) a compound of formula 1 or 2 wherein $R_5$ is absent, i) is converted with a compound $R_5$-X, wherein $R_5$ is alkyl, and X is a group which may be replaced by a nucleophile; or ii) is oxidised with a suitable oxidant.

European Patent Office

# EUROPEAN SEARCH REPORT

Application Number

EP 88 20 2856

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 217 454 (J.G. BERGER)<br>* Columns 1-4 (nrs. VI,VIa) *<br>--- | 1 | C 07 D 471/16<br>A 61 K 31/395<br>C 07 D 521/00 |
| A | US-A-3 299 078 (I.J. PACHTER)<br>* Columns 1-6 *<br>----- | 1,2 | |
| | | | **TECHNICAL FIELDS SEARCHED (Int. Cl.4)** |
| | | | C 07 D 471/00 |

The present search report has been drawn up for all claims

| Place of search | Date of completion of the search | Examiner |
|---|---|---|
| THE HAGUE | 23-03-1989 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)